(19)

**Europäisches Patentamt**
**European Patent Office**
**Office européen des brevets**

(11) **EP 4 350 699 A1**

(12) **EUROPEAN PATENT APPLICATION**

(43) Date of publication:
**10.04.2024  Bulletin 2024/15**

(21) Application number: **22382921.9**

(22) Date of filing: **03.10.2022**

(51) International Patent Classification (IPC):
***G16C 20/20*** *(2019.01)*

(52) Cooperative Patent Classification (CPC):
**G16C 20/20;** G01N 30/86; G16B 40/10;
G16C 20/80; H01J 49/0036

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Olobion, S.L.**
**08028 Barcelona (ES)**

(72) Inventors:
• **BONINI, PAOLO**
 **08028 BARCELONA (ES)**
• **MEHTA, SAJJAN SINGH**
 **08028 BARCELONA (ES)**

(74) Representative: **Isern Patentes y Marcas S.L.**
 **Avda. Diagonal, 463 Bis, 2°**
 **08036 Barcelona (ES)**

(54) **METHOD FOR METABOLOMIC PROFILING OF A HOLOBIONT**

(57) The invention relates to a method for metabolomic profiling of a holobion using Liquid Chromatography-Mass Spectrometry.

EP 4 350 699 A1

## Description

The present invention relates to a method for metabolomic profiling of a holobiont using Liquid Chromatography-Mass Spectrometry.

## BACKGROUND

The holobiont, a term coined in 1990 by Margulis (Words as battle cries--symbiogene-sis and the new field of endocytobiology. Biocience. 1990 Oct; 40(9):673-7), is a biological concept that extends the notion of an individual and isolated organism to include its associated community of microorganisms that exists on or around it. These microorganisms, also referred to as the microbiota, interact with their host in numerous ways, ranging from symbiotic to parasitic or pathogenic. For instance, plants have intricate, bidirectional interactions with soil microbials, and the human gut microbiome influences the body far beyond the digestive system in which it resides.

The combination of the host organism and its associated microbiota constitutes a discrete ecological unit called a holobiont, and this framework enables exploration into the complex host-microbiota interactions which are increasingly being recognized as highly influential to the development, growth, and overall health of the host.

Plant, animals, fungi, and bacteria all produce metabolites, small molecules involved in metabolic processes. Metabolomics is the scientific field focused on the comprehensive analysis of metabolites and the chemical processes in which they interact with biological systems. Metabolomics studies are generally performed using untargeted or targeted approaches.

Untargeted metabolomics attempts to comprehensively characterize metabolites with multiple degrees of precision and also to measure the variation in metabolite levels between two or more experimental conditions each with multiple samples. Targeted metabolomics uses a well-defined set of metabolites to directly quantify their abundances in a single sample.

Metabolites have various functions in biological processes including serving as fuel, structure, signaling, stimulatory and inhibitory effects on enzymes, defense, and interactions with other organisms.

The synthesis of particular secondary metabolites in the plant roots and leaves can be influenced by different factors including lack of nutrients, cold stress, heat stress, insect presence, pathogenic microbials, and beneficial fungi or plant promoting bacteria.

For instance, metabolites can be produced to deter insects, to combat fungal or bacterial pathogens, or to bind to biologically unavailable metals in the soil in order to improve their solubility. Recent investigations also reveal the plant's ability to modulate root microbiota through metabolite production, encouraging growth of or repelling microbes which in turn can help to alleviate environmental stresses.

The soil is not just the support of the plant. It is a complex world where millions of microbial, insects, algae, archea and others life forms live. All of these organisms can exist in a balance, but constantly in change due to the climate and the plant exudates. Most of the time this equilibrium is not optimal for plant growth and yield. Being able to analyse and understand it empower to take the correct decisions.

In the same way, plant leaves support microbial life and have associated microbiota that can produce important compounds for protecting plants against pathogens and increasing plant growth through hormone production. It is important to understand the balance in the holobiont and how it can be altered and improved to serve the plant better.

These techniques can be else applied to human or animal skin, urine, and stool samples. The human skin hosts microbials that interact with the human lipidome and metabolome, modifying and transforming it. On the other hand, it is known that the human bowel contains a high variety of microbials that have implication in health and psychology. With the inventive method it is also possible to analyze the metabolic active microbials and correlate them to specific health problems.

## SUMMARY OF THE INVENTION

In a first aspect, the present invention relates to a method for metabolomic profiling of a holobiont, said method comprising the steps of:

(a) subjecting a sample from a holobiont to chromatography-tandem mass spectrometry to acquire sets of experimental mass spectra;
(b) matching said sets of experimental mass spectra against library mass spectra of known metabolites to identify likely candidate metabolites in said holobiont sample;
(c) calculating predicted mass spectrometry fragmentation patterns and/or predicted chromatographic retention times and/or predicted collision cross sections (CCS) for likely candidate metabolites identified in step (b) using machine learning models;

(d) matching the sets of experimental mass spectra acquired in step (a) against the predicted mass spectrometry fragmentation patterns and/or predicted chromatographic retention times and/or predicted collision cross sections calculated in step (c); and

(e) using a similarity metric to quantify the relationship between the experimental mass spectra and the predicted mass spectrometry fragmentation patterns and/or predicted chromatographic retention times to identify metabolites present in the holobiont sample.

In a second aspect, the present invention uses in step (a) above as the chromatography liquid chromatography, for example, reversed-phase (RPLC) or hydrophilic interaction chromatography (HILIC), and the mass spectrometry is selected from the group consisting of electron impact ionization (EI), electrospray ionization (ESI), atmospheric pressure chemical ionization (APCI), or matrix-assisted laser desorption/ionization (MALDI) mass spectrometry.

In a third aspect of the invention, two mass analyzers selected from the group consisting of a quadrupole mass analyzer, an ion trap mass analyzer, an orbitrap mass analyzer, a time-of-flight mass analyzer, and a Fourier transform mass analyzer are arranged to perform tandem mass spectroscopy.

In a fourth aspect of the present invention, the predicted mass spectrometry fragmentation patterns of step (c) are calculated using the bond dissociation approach for known metabolites generating two fragments per split bond as well as using established rules for known recombinations of molecular fragments.

In a fifth aspect of the present invention, the predicted chromatographic retention times and/or collision cross sections of step (c) are calculated using quantitative structure-retention relationships from predicted physical and chemical characteristics of known metabolites comprising atomic and bond properties, chemical properties of individual atoms or functional groups, measures of molecular properties such as lipophilicity or hydrophobicity, and geometrical and topological properties of the metabolites.

In a sixth aspect of the present invention, the machine learning model used in step (c) is selected from the group consisting of XGBoost, BRNN, Random Forest, LightGBM, Deep Neural Networks (using Keras, PyTorch, or FastAi), CatBoost, and K-Nearest Neighbors, or a general bagging (bootstrap aggregation, a method of improving model accuracy and avoiding overfitting) or stacking (a method of combining predictions from multiple machine learning models) ensemble of a subset of the models.

In a seventh aspect of the present invention, the identified metabolites of the holobiont sample are matched against libraries of biomarker metabolites to identify individual life forms of the holobiont. These libraries can be developed internally or public libraries can be used, for example, https://www.npatlas.org/ or https://lotus.naturalproducts.net/ or https://pmn.plantcyc.org/ or https://pubchem.ncbi.nlm.nih.gov/.

In an eighth aspect of the present invention, the individual life forms of the holobiont sample comprise plant species, fungal species, bacterial species, or other eukaryotic life species.

In a ninth aspect of the present invention, scaled and summed peak intensities of the experimental mass spectra are used to calculate holobiont balance factors, wherein the holobiont balance factors are selected, for example, from the group consisting of ratios showing the relative abundance of plant vs. fungal metabolites, plant vs. bacterial metabolites, and fungal vs. bacterial metabolites.

In a tenth aspect of the present invention, for any set of identified metabolites, a measure of chemical diversity $\phi$ is calculated in one of three ways.

$$\emptyset_{Jaccard} = \sum_{a,b \in M} \sum_{i} w_a w_b (1 - F_i(a) \wedge F_i(b))$$

$$\emptyset_{Shannon} = -\sum_{k \in M} p_k \ln(p_k)$$

$$\emptyset_{Simpson} = \sum_{k \in M} p_k^2$$

wherein $M$ is the set of number of identified metabolites; $w_k$ is the weight for a given metabolite $k$ and is a transformed chromatographic peak height or area using any combination of log transformation, intensity scaling, and correction for ionizability; $p_k$ is the metabolite weights converted to a probability distribution for a given metabolite $k$; and the $F_i$ are

chemical fingerprinting functions, wherein the chemical fingerprinting functions, for example, comprise one or more of Morgan/Circular, Avalon, MACCS, PubChem, Topological-Torsion, CDK, and/or RDKit fingerprints. The chemical fingerprinting functions $F_i$ yield bit vectors, which are compared using the bitwise/logical AND operator represented by $\Lambda$.

In an eleventh aspect of the present invention, all identified metabolites are displayed in a graph network where node length depends on chemical similarity. Chemical nodes are represented with shorter edge lengths if they are more similar and longer if they are dissimilar.

In a twelfth aspect of the present invention, the identified individual life forms of the holobiont are grouped and highlighted into beneficial and pathogenic life forms.

In a thirteenth aspect of the present invention, for any set of identified metabolites a plant defense-pathogen ratio as an indicator of the immunity or susceptibility of a plant to invading pathogens or other organisms is calculated according to the formula:

$$\frac{\psi_{int,PlantDefense}}{\psi_{int,Pathogen}}$$

wherein for a given set of metabolites M and weight $w_k$ for a given metabolite k

$$\psi_{int} = \sum_{k \in M} w_k$$

$\psi_{int,PlantDefense}$ is the Plant Defense Normalized Intensity of the set of identified metabolites that defend plants against pathogens and $\psi_{int,Pathogen}$ is the Plant Pathogen Normalized Intensity of the set of identified metabolites that are pathogenic to plants.

This summary of the invention does not necessarily describe all features and/or all aspects of the present invention. Other embodiments will become apparent from a review of the ensuing detailed description.

## DETAILED DESCRIPTION

### Definitions

In the following, the invention is described in more detail with reference to the Figures. The described specific embodiments of the invention, examples, or results are, however, intended for illustration only and should not be construed to limit the scope of the invention as indicated by the appended claims in any way.

It is to be understood that this invention is not limited to the particular methodology, protocols, and reagents described herein as these may vary. It is also to be understood that the terminology used herein is to describe particular embodiments only, and is not intended to limit the scope of the present invention which will be limited only by the appended claims. Unless defined otherwise, all technical and scientific terms used herein have the same meanings as commonly understood by one of ordinary skill in the art.

Each of the documents cited in this specification (including all patents, patent applications, scientific publications, manufacturer's specifications, instructions, etc.), whether supra or infra, is hereby incorporated by reference in its entirety. In the event of a conflict between the definitions or teachings of such incorporated references and definitions or teachings recited in the present specification, the text of the present specification takes precedence.

The term "comprising" or variations thereof such as "comprise(s)" according to the present invention (especially in the context of the claims) is to be construed as an open-ended term or non-exclusive inclusion, respectively (i.e., meaning "including, but not limited to,") unless otherwise noted.

The term "comprising" shall encompass and include the more restrictive terms "consisting essentially of' or "comprising substantially", and "consisting of'.

In the case of chemical compounds or compositions, the terms "consisting essentially of' or "comprising substantially" mean that specific further components can be present, namely those not materially affecting the essential characteristics of the compound or composition, e.g., unavoidable impurities.

The terms "a", "an", and "the" as used herein in the context of describing the invention (especially in the context of the claims) should be read and understood to include at least one element or component, respectively, and are to be construed to cover both the singular and the plural, unless otherwise indicated herein or clearly contradicted by context.

In addition, unless expressly stated to the contrary, the term "or" refers to an inclusive "or" and not to an exclusive "or" (i.e., meaning "and/or").

All numeric values are herein assumed to be modified by the term "about", whether or not explicitly indicated. Recitation of ranges of values herein is merely intended to serve as a shorthand method of referring individually to each separate value falling within the range, unless otherwise indicated herein, and each separate value is incorporated into the specification as if it were individually recited herein.

The use of terms "for example", "e.g.", "such as", or variations thereof is intended merely to better illuminate the invention and does not pose a limitation on the scope of the invention unless otherwise claimed. These terms should be interpreted to mean "but not limited to" or "without limitation".

The term "selected from the group consisting of' means that one or more members of the group may be selected, and in any combination.

The terms "we" and "our" used in the following mean the present inventors.

## EMBODIMENTS OF THE INVENTION

We have developed a novel analysis workflow for deriving insight into the interactions between an organism and its microbial environment (holobiont) using metabolomics.

Analyses of bacteria and fungi in soil samples are typically performed using metagenomics, which can detect microbials and identify species differences with high accuracy. However, running samples is comparatively expensive, many samples need to be run at once for the highest cost effectiveness, and the number of reads can vary significantly by sample and run. Additionally, there is no global set of primers, which requires running the same sample multiple times to obtain fungal and bacterial DNA amplification and sequencing.

Metabolomics using mass spectrometry has the advantage of being more time-efficient and inexpensive to run for individual samples; however, typical untargeted metabolomics experiments are designed to compare the relative abundances of metabolites using univariate or multivariate statistical analysis between at least two sets of at least 4 samples, where the sample sets possess some difference in treatment or preparation.

Our invention overcomes these limitations of untargeted metabolomics, allowing data generation from a single sample by using

1) the presence of significant metabolites to recognize metabolic indicators of host organism and identify microbials in the host's environment,

2) specific ratios between molecules of different biological origins (plant, fungi, bacteria, etc.) to provide insight into the associated life systems.

Using our workflow, we can reveal information about not only the presence but also the activity of microbials, about chemical interactions between the microbials and the host, and about the overall health of the holobiont system.

We have developed techniques to map discovered metabolites to organisms, enabling us to identify metabolically active microbials while ignoring inert or inactive microbes that are not impacting plant, animal, or human health.

We define active microbials as the ones that are metabolically producing specific compounds that can be linked to their taxonomy. Metagenomics analyze DNA fragments at this can be obtained also from dormient spores or inactive cells.

Mapping of metabolites to microbials is performed using a compilation of literature knowledge and chemical databases to assign known organism origins to metabolites. When comparing soil samples over time we model the change of the metabolite intensities over time to determine the activity of associated microbials. Similarly, when comparing soil sample along with a sample of leaves from the same field, we can use the historical.

We developed a fully automated workflow based on artificial intelligence. This workflow takes the mass spectrometry data coming from high resolution mass spectrometry instrument along with study design metadata (which includes sample origin, matrix information, experimental groups, LC-MS experimental conditions, and sample injection order) and transforms it into an interactive report containing general statistics, chemical enrichment analysis, pathway enrichment analysis, MS/MS and chemical networking analysis, and annotations of microbial taxonomy and function.

An extraction using a mixture of organic solvents is used; specifically we found that 70% methanol or ethanol with 30% of water is the most effective to the degradation microbials cell and extract the molecules present inside. A liquid chromatographic system coupled to a high-resolution mass spectrometer (LC-MS/MS) is used for data acquisition.

Feature detection is performed by identifying Gaussian peaks in extracted ion chromatograms at the MS1 level (summed ion chromatograms for a limited mass range, iterated over all available masses) and matching them to acquired MS/MS spectra. This step can be performed using numerous algorithmic approaches, and we utilize MS-DIAL 4.9 which is integrated into our workflow.

For metabolite identification, we apply a multi-stage feature annotation process that utilizes mass spectrometry library data for high-reliability MS/MS similarity annotations and chemical databases of known natural products for in-silico MS/MS prediction and annotation.

The first step of the annotation pipeline is to search all acquired MS/MS spectra against a subset of the authentic MS/MS libraries whose mass spectra corresponds to known molecules present in plant and microbial life. This search utilizes a similarity metric or combinations of similarity measures, for example the dot product score, cosine similarity, Jaccard similarity, or spectral entropy, to quantify the relationship between the experimental and library mass spectra.

Next, an in-silico annotation approach utilizing machine learning is applied using chemical databases. Several in-silico identification approaches exist for small molecule mass spectrometry, including ChemDistiller which combines molecular bond-breaking fragmentation prediction with chemical fingerprinting to reliably match chemical structures to MS/MS spectra. This in-silico approach is repeated on increasingly broad chemical databases to maximize annotation coverage.

All compounds in the MS/MS libraries and chemical databases are assigned predicted retention times and/or predicted collision cross sections which are calculated using machine learning, and retention time filtering and/or collision cross section filtering is applied at each annotation step. While retention time prediction is well-established in proteomics, the diversity of small molecules in biological samples requires more sophisticated approaches for metabolomics and natural products databases. We utilize a tool called Retip that builds ensemble machine learning models to predict retention times from validated datasets of authentic standards using the Mordred chemical descriptor set as training features. An analogous technique is used for the prediction of collision cross sections. After feature annotation, metabolite metadata are supplemented with chemical taxonomical classification from ClassyFire and NPClassifier, which are public machine learning-based tools that assign chemical taxonomies to chemical structures. Each metabolite is then associated with a chemical origin category based on its presence in an internal common-life chemical database or based on literature-derived organism associations in public databases including but not limited to: PlantCyc (https://plantcyc.org), MetaCyc (https://metacyc.org), NPAtlas (https://npatlas.org) and LOTUS (https://lotus.natu-ralproducts.net).

We also developed a fast-screening procedure to find species-specific compounds using pure culture fungi or bacterial strains. We grow the microbes in plates or tubes, extract the metabolites in a solution 70:30 methanol water and analyze with mass spectrometry.

MS/MS feature detection and putative annotation is performed for each microbial sample, after which all data is combined into a tabular format. After discarding common-life metabolites and other ubiquitous small molecules, we identify biomarker candidates which are specific compounds uniquely associated with individual microbials. These are used to improve biological origin mapping pipeline.

The category options include common life, animals, plants, fungi, bacteria, microbia, other life forms, or unclassified.

Identified metabolites can then be partitioned by chemical origin, and from this a graphical biological origin figure is produced.

In addition, scaled and summed intensities are used to calculate holobiont balance indicators, ratios showing the relative abundance of plant vs. fungal, plant vs. bacterial, and fungal metabolites vs. bacterial metabolites. In the case of animal samples, we also calculate the ratio including animal origin compounds.

We then further categorize metabolites based on their structure and function. Secondary metabolites associated with plant defense are identified based on known pathogen attack response, other metabolites are grouped by their known antimicrobial properties, and finally identified siderophore and peptiabols are highlighted due to their beneficial nutrient uptake and antibiotic properties, respectively.

Metabolites directly associated to notable fungi and bacteria are listed based on the microbe's advantageous or pathogenic properties to plant or animal life.

Finally, we developed a measure of chemical diversity (phi diversity). For each identified metabolite, chemical fingerprints (a combination of fingerprints available in the RDKit chemical library including but not limited to Morgan/Circular and Avalon fingerprints) are computed, and structural dissimilarity is calculated between all unique pairs of metabolites using the Tanimoto similarity metric. The dissimilarities are aggregated for all metabolites as well as for subsets corresponding to chemical classes associated with microbial groups, and these totaled dissimilarities are presented as φ diversity.

Chemical diversity $\phi$ is calculated in one of three ways.

$$\emptyset_{Jaccard} = \sum_{a,b \in M} \sum_{i} w_a w_b (1 - F_i(a) \wedge F_i(b))$$

$$\emptyset_{Shannon} = -\sum_{k \in M} p_k \ln(p_k)$$

$$\emptyset_{Simpson} = \sum_{k \in M} p_k^2$$

wherein $M$ is the set of number of identified metabolites; $w_k$ is the weight for a given metabolite $k$ and is a transformed chromatographic peak height or area; $p_k$ is a set of metabolite weights converted to a probability distribution for a given metabolite $k$; and the $F_i$ are chemical fingerprinting functions.

Using the data produced in our workflow, we are able to predict the future yield of the field using regression models based on machine learning.

Although plants lack an immune system as complex as animal life, they have evolved a number of chemical and protein-based defense mechanisms that can provide various degrees of protection against invading pathogens and other organisms.

The degree to which plants can defend itself against external conditions range from immunity (the complete lack of any disease symptoms) to highly resistant (some disease symptoms) to highly susceptible (significant disease symptoms).

Plants exposed to the same pathogen may manifest disease symptoms depending how strongly plant defenses are triggered.

Plants produce two types of chemical products. Primary metabolites are compounds related to plant metabolism, growth and development. Secondary metabolites are compounds that help the plant to interact with its environment, respond to environmental stresses, and defend against pathogens and other organisms.

We have built a library of plant defense compounds based on compounds reported in existing literature and on molecules belonging to chemical families related to defense functions. The compounds identified in the described workflow are compared to the plant defense library and labeled accordingly. The indicator "Plant Defense Number" is calculated by counting the number of compounds that match the plant defense library.

The indicator "Plant Defense Normalized Intensity" is calculated by summing each plant defense compound chromatographic peak signal which are normalized by using any combination of one or more of log transformation, intensity scaling, and linear regressions to correct for molecular ionizability.

The index $\psi$ (psi) is used as a measure of abundance of a particular set of metabolites. For example, for a given set of metabolites $M$, $\psi_n$ is the number of metabolites observed above an observation threshold epsilon (0 by default):

$$\psi_n(\epsilon) = \sum_{k \in M} I(w_k > \epsilon)$$

where $I$ is the identity function and returns 1 if the condition is met (i.e., a metabolite is detected) and 0 otherwise, and $w_k$ is the weight for a given metabolite $k$ and is a transformed chromatographic peak height or area using any combination of one or more of log transformation, intensity scaling, and linear regressions to correct for molecular ionizability.

Therefore, $\psi_{n,PlantDefense}$ is the Plant Defense Number. $\psi_{int}$ uses normalized intensity to determine abundance:

$$\psi_{int} = \sum_{k \in M} w_k$$

Thus, $\psi_{int,PlantDefense}$ is the Plant Defense Normalized Intensity.

From this, we can calculate the plant defense-pathogen ratio, which is an indicator of the immunity or susceptibility of a plant to invading pathogens or other organisms:

$$\frac{\psi_{int,PlantDefense}}{\psi_{int,Pathogen}}$$

The values of the plant defense-pathogen indicator may differ by crop type, environmental conditions, and disease type.

## BRIEF DESCRIPTION OF THE FIGURES

The following Figures are merely illustrative of the present invention and should not be construed to limit the scope of the invention as indicated by the appended claims in any way.

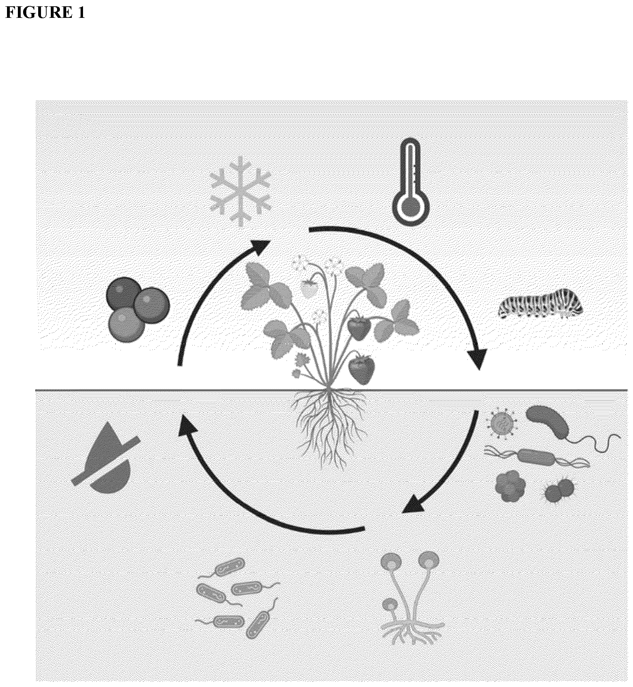

> **FIGURE 1** exemplifies a plant holobiont.
> **FiGURE 2** shows the origin of chemical diversity in a soil sample obtained with the inventive method. Hongo = Fungi, Planta = Plants, Otre vida = other life forms, Sin clasificar = unclassified
> **FIGURE 3** shows the identified metabolite classes in a soil sample
> **FIGURE 4** shows a chemical similarity map.

## EXAMPLES

The examples given below are for illustrative purposes only and do not limit the invention described above in any way.

Materials and Methods

Prediction of chromatographic retention times

To predict chromatographic retention times for likely candidate metabolites we utilized three publicly available LC-MS spectra libraries for retention time prediction model development and validation. For development of a hydrophilic interaction chromatography (HILIC) data set we utilized the MassBank of North America (MoNA) database (http://massbank.us/). The HILIC data set contained a total of 970 compounds, including MS/MS spectra and retention time, with methods given in the MoNA database as using a Waters Acquity UPLC-BEH Amide column (150 mm $\times$ 2.1 mm; 1.7 $\mu$m) coupled to an Acquity UPLC BEH Amide VanGuard precolumn ($5 \times 2.1$ mm; 1.7 $\mu$m). The column was maintained at 45 °C with a flow rate of 0.4 mL/min. The mobile phases consisted of (A) water with ammonium formate (10 mM) and formic acid (0.125%) and (B) acetonitrile:water (95:5, v/v) with ammonium formate (10 mM) and formic acid (0.125%). The separation was conducted under the following gradient: 0 min 100% B; 0-2 min 100% B; 2-7.7 min 70% B; 7.7-9.5 min 40% B; 9.5-10.25 min 30% B; 10.25-12.75 min 100% B; 12.75-17 min 100% B. We used the "Pathogen Box" (https://www.mmv.org/mmv-open) data set measured using the same Waters BEH Amide HILIC column as the external validation set. Chemical diversity and mass spectra can be downloaded from the MoNA database.

For the reversed-phase liquid chromatograph (RPLC) data set, we used the RIKEN plant specialized metabolome annotation (PlaSMA) database (http://plasma.riken.jp), created with fully labeled $^{13}$C plants and enriched metabolites, which were measured with a Waters Acquity ultraperformance liquid chromatography (UPLC) ethylene-bridged hybrid (BEH) C18 column (100 mm $\times$ 2.1 mm; 1.7 $\mu$m particle diameter), maintained at 40 °C. The mobile phases consisted of (A) water including 0.1 % formic acid and solvent (B) acetonitrile including 0.1% formic acid. The separation was conducted under the following gradient: 0.5% (B) at 0 min; 0.5% (B) at 0.1 min; 80% (B) at 10 min; 99.5% (B) at 10.1 min; 99.5% (B) at 12.0 min; 0.5% (B) at 12.1 min, isocratic until 15.0 min. A flow gradient was employed from 0.3 to 0.4 mL/min. For the case study, we utilized public data sets containing BioRec (now BioIVT) human blood plasma MS/MS data downloaded from http://mctabolomicsworkbcnch.org with accession ID ST001154. Compounds were annotated using the Fiehn Lab HILIC experimental MS/MS spectral library. These 143 identified metabolites ("true positives") were used as "test set" for HILIC retention time prediction and removed from the overall HILIC libraries downloaded from MoNA. Residual HILIC library entries were used as training set molecules.

Computational Methods-Structure Standardization and Cleaning.

Compound structures were curated with the ChemAxon standardizer to remove salts and metal-containing compounds. Simplified Molecular Input Line Entry System (SMILES) codes that were not compatible with the R platform-based Chemistry Development Kit (rCDK) and ChemAxon and OpenBabel toolkits were excluded or reformatted. Whenever possible, we also used the structure-data file (*.sdf) format to avoid conversion errors.

## Calculating Chemical Compound Descriptors

Chemical descriptors are more interpretable than structure fingerprints. We utilized multiple descriptor packages, including the CDK, Padel, Dragon 7, and alvaDesc (Kode Chemoinformatics srl, Italy) as weil as ChemAxon. For the final implementation, we used CDK descriptors that can be publicly distributed as a software package in rCDK. The initial SMILES data processing was implemented as a parsing function in the Retip function getCD(). This code relies on the "rcdk" package (version 3.4.7.1), an R interface to the CDK. Compounds that failed during descriptor calculation were automatically removed. In total, 286 chemical descriptors were computed for each library compound. After the SMILES code was exported from different libraries, we generated 2D coordinates based on the connectivity data. For our current version of Retip, we utilized 2D-based descriptors due to the computational overhead for 3D-optimized structures. SMILES codes were converted to the ChemAxon Extended SMILES (*.cxsmiles) format to store additional atom properties and coordinates. Explicit hydrogens were added for correct representations and accurate atomic and enhanced atomic partition coefficient (Alog and XlogP, respectively) calculations. Finally, we added ChemAxon (https://chemaxon.com/) pK values, including the acidic ($pK_{a1}$, $pK_{a2}$) and basic (pKb1 and $pK_{b2}$) pK values, because initial investigations showed that these descriptors may improve HILIC predictions. The alvaDesc descriptor software was used for visual inspections molecular weight histograms, logP distributions, and multivariate inspections (principal component analysis, PCA).

## Machine Learning Models

Retention time prediction utilizing chemical descriptors can be described as a regression problem. We utilized root-mean-square errors (RMSEs) as a loss function for resulting regression models by calculating the minimized residuals between observed and predicted values. We used correlation $R^2$ values between observed and predicted retention times to indicate linear relationships and for global generalization of the prediction set. For all models except for Keras, 10-fold cross-validation was employed. In Keras, the internal function validation_split was set to 0.2, instead.

Parameter tuning is an essential step for good model performance. Tuning parameters can include random searches or grid searches of the parameter space. We used five independent regression models including parameter tuning:

(1) XGBoost performs gradient-boosting for regression and classification problems. We implemented automatic grid search tuning for the parameters nrounds, max_ depth, and eta, while the fixed parameters were gamma, colsample_bytree, subsample, and min_child_weight.

(2) Keras is a high-abstraction layer available for GPU and CPU processing for deep learning and neural networks, using TensorFlow, the Microsoft Cognitive Toolkit, and Theano libraries. Data were centered and scaled. We automatically tuned the dense _ unit, epochs, and dropout parameters; other parameters such as batch _size and learning_rate were manually tuned.

(3) The light gradient-boosting machine (LightGBM) is known for its high efficiency and low RAM usage. It can efficiently process millions of rows in parallel. For parameter optimization, Retip automatically searches for the optimal nrounds parameter based on the best iter value in the cross-validation model. Other model values, such as regressions L1 and L2 regularization, the learning rate, eval freq, metric, early_stopping_rounds, maximum depth, maximum leaf, and maximum bin were manually tuned to identify the best values and deal with overfitting.

(4) The random forest (RF) algorithm is one of the most popular algorithms in machine learning. We tuned the mtry parameter, which descnbes the number of variables that are sampled as candidates for each split.

(5) We tuned the number of neurons for the Bayesian-regularized neural network (BRNN), an algorithm that uses Bayesian regularization for feedforward neural networks.

## Retip R Package Functions

Functions of the Retip package are explained in detail in the online R package documentation and the GitHub-hosted Web site (https://www.retip.app/). Retip enables a complete workflow from experimental retention time data to a final deployable prediction model. The prepare.wizard() function activates the parallel computation inside Retip. The getCD() function is utilized to compute chemical descriptors. The cesc() function is needed to center and scale the data set, especially for neural network predictions. The chem.space() function plots molecules based on chemical similarity in principal components analysis. Two libraries can be superimposed, for example, a training and a test compound library. The proc.data() function handles non-existent values and low-variance columns. Machine learning models use fitting functions with parameter optimizations (i.e., fit.rf fit.brnn, fit.keras, fit.xgboost, and fit.lightgbm). The get.score() function calculates model statistics, including RMSE, $R^2$, MAE, and 95% confidence intervals. The plot.model() function plots retention time error distributions. The RT.spell() function predicts the retention times of user-uploaded models. The prep.mona() function allows integration with the freely available MoNA interface. The add.rt.mona() function was employed to add RT information in the mass search format spectral files (*.msp) that can be utilized with National Institute of Standards and Technology (NIST)-compatible MS/MS search software.

Integration with Independent Mass Spectrometry Software

To integrate retention time prediction results into independent software packages, we provide the RT.export() function in the Retip R package. This function enables the use of retention time filters in packages such as MS-DIAL, MSFINDER, the Agilent MassHunter Suite, and Waters and ThermoFisher Scientific software. The R package documentation with example data sets is provided at the Retip GitHub code repository (https://www.retip.app). Retip supports the open-source software packages MS-DIAL and MS-FINDER with msp formatted MS/MS spectra. For MS-FINDER, use of Retip was newly developed for scoring or filtering structure candidates by retention time similarity using Gaussian functions and RT tolerances.

Retip Package Versions and Hardware

Retip was built as a package in R (3.5.3) using R Studio 1.1.143. The R dependencies are caret (6.0-81), ggplot2 (3.1.0), rcdk (3.4.7.1), doParallel (1.0.14), keras (2.2.4.9), stringi (1.4.3), xgboost (0.82.1), brnn (0.7), and lightgbm (2.2.3). The hardware employed was an HP Zbook 15 G5 mobile workstation with an Intel(R) Xeon(R) E-2186 M CPU at 2.90 GHz with 6 cores, 12 logical processors, and 64 GB of RAM and running Windows 10 Pro 64-bit.

Results

Keras outperformed other machine learning algorithms in the test set with minimum overfitting, verified by small error differences between training, test, and validation sets. Keras yielded a mean absolute error of 0.78 min for HILIC and 0.57 min for RPLC. Retip is integrated into the mass spectrometry software tools MS-DIAL and MS-FINDER, allowing a complete compound annotation workflow. In a test application on mouse blood plasma samples, we found a 68% reduction in the number of candidate structures when searching all isomers in MS-FINDER compound identification software. Retention time prediction increases the identification rate in liquid chromatography and subsequently leads to an improved biological interpretation of metabolomics data.

Example 1: Soil sample holobiont analysis

The presence of metabolites and peptaibols in a soil sample was determined by liquid chromatography followed by tandem mass spectrometry with an electrospray ionization source (UHPLC-ESI-MSMS-TOF).

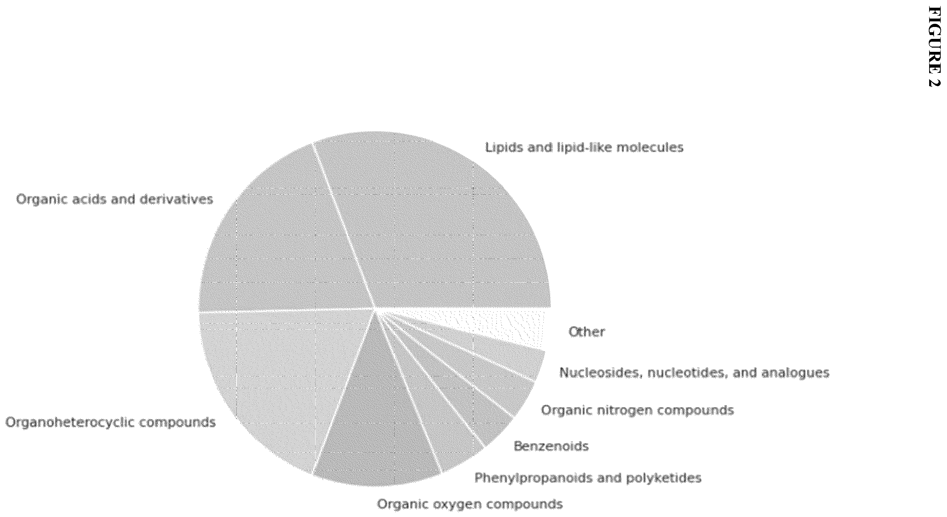

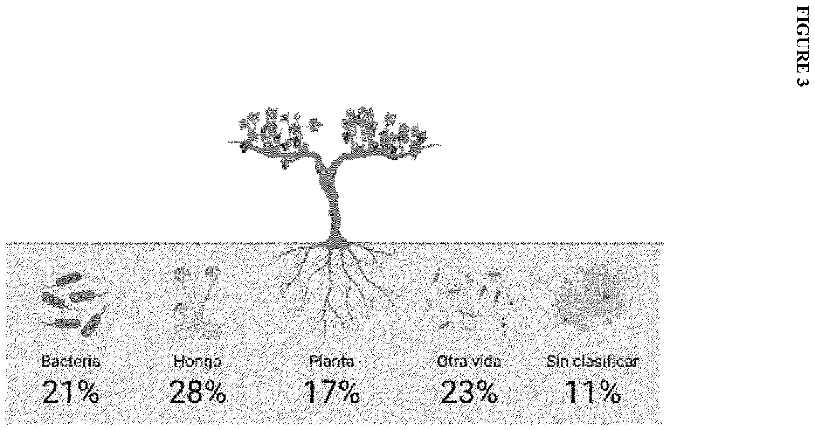

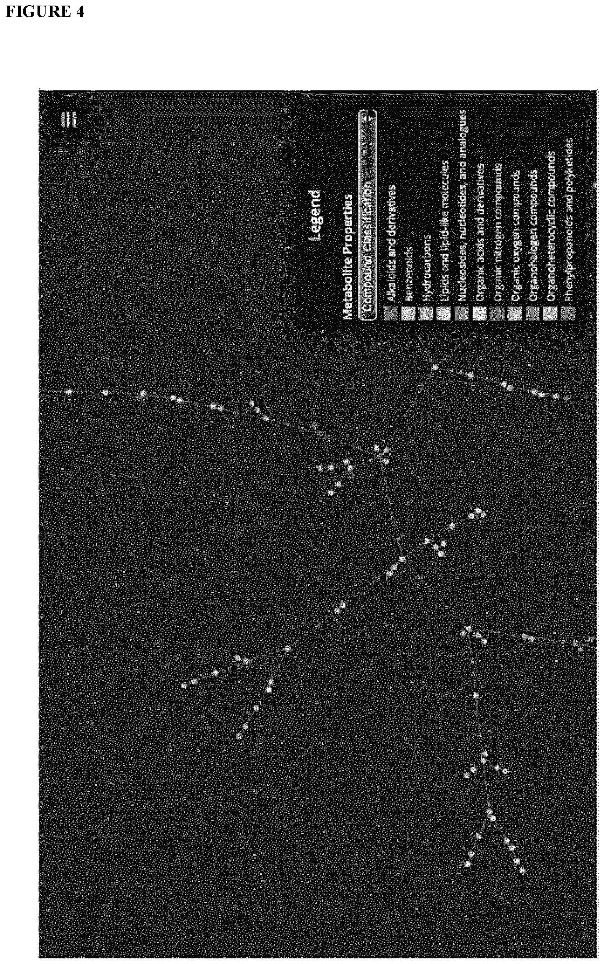

FIGURE 2 shows the origin of chemical diversity in the soil sample obtained with the inventive method. Hongo = Fungi, Planta = Plants, Otre vida = other life forms, Sin clasificar = unclassified. FIGURE 3 shows the identified metabolite classes in the soil sample, and FIGURE 4 shows a chemical similarity map.
The calculates holobiont balance is:

Ratio plants/fungi: 0.65; Ratio plants/bacteria: 0.86; Ratio fungi/bacteria: 1.32
Plant defense metabolites: 3, peptaibol metabolites 6; siderophores metabolites: 11
Identified beneficial microbiota: *Metarhizium, Nodularia, Penicillium, Streptomyces, Streptoverticillium*
Pathogenic microbiota: *Aspergillus, Fusarium, Pestalotiopsis*

While certain representative embodiments and details have been shown to illustrate the present invention, it will be apparent to those skilled in this art that various changes and modifications can be made and that, within the scope of the appended claims, the invention may be practiced otherwise than as specifically described and claimed.

**Claims**

1. A method for metabolomic profiling of a holobiont, said method comprising the steps of:

(a) subjecting a sample from a holobiont to chromatography-tandem mass spectrometry to acquire sets of experimental mass spectra;

(b) matching said sets of experimental mass spectra against library mass spectra of known metabolites to identify likely candidate metabolites in said holobiont sample;

(c) calculating predicted mass spectrometry fragmentation patterns and/or predicted chromatographic retention times and/or predicted collision cross sections for likely candidate metabolites identified in step (b) using machine learning models;

(d) matching the sets of experimental mass spectra acquired in step (a) against the predicted mass spectrometry fragmentation patterns and/or predicted chromatographic retention times and/or predicted collision cross sections calculated in step (c); and

(e) using a similarity metric to quantify the relationship between the experimental mass spectra and the predicted mass spectrometry fragmentation patterns and/or predicted chromatographic retention times to identify metabolites present in the holobiont sample.

2. The method according to claim 1, wherein in step (a) the chromatography is liquid chromatography, and the mass spectrometry is selected from the group consisting of electron impact ionization (EI), electrospray ionization (ESI), atmospheric pressure chemical ionization (APCI), or matrix-assisted laser desorption/ionization (MALDI) mass spectrometry.

3. The method according to claims 1 or 2, wherein two mass analyzers selected from the group consisting of a quadrupole mass analyzer, an ion trap mass analyzer, an orbitrap mass analyzer, a time-of-flight mass analyzer, and a Fourier transform mass analyser are arranged to perform tandem mass spectroscopy.

4. The method according to claims 2 or 3, wherein the liquid chromatography is reversed-phase or hydrophilic interaction chromatography.

5. The method according to any one of the preceding claims, wherein in step (c) the predicted mass spectrometry fragmentation patterns are calculated using the bond dissociation approach for known metabolites generating two fragments per split bond as well as using established rules for known recombinations of molecular fragments.

6. The method according to any one of the preceding claims, wherein in step (c) the predicted chromatographic retention times and/or predicted collision cross sections are calculated using quantitative structure-retention relationships from predicted physical and chemical characteristics of known metabolites comprising atomic and bond properties, chemical properties of individual atoms or functional groups, measures of molecular properties such as lipophilicity or hydrophobicity, and geometrical and topological properties of the metabolites.

7. The method according to any one of the preceding claims, wherein in step (c) the machine learning model is selected from the group consisting of XGBoost, BRNN, Random Forest, LightGBM, Deep Neural Networks, CatBoost, and K-Nearest Neighbors, or a general bagging or stacking ensemble of a subset of the models.

8. The method according to any one of the preceding claims, wherein the identified metabolites of the holobiont sample are matched against libraries of biomarker metabolites to identify individual life forms of the holobiont.

9. The method according to claim 8, wherein the individual life forms of the holobiont sample comprise prokaryotic and/or eukaryotic life species such as plant species, fungal species, bacterial species, and wherein holobiont balance factors selected from the group consisting of ratios showing the relative abundance of plant vs. fungal metabolites, plant vs. bacterial metabolites, and fungal vs. bacterial metabolites are calculated, where abundance corresponds to scaled and summed chromatographic peak intensities of the identified metabolites.

10. The method according to any one of the preceding claims, wherein for any set of identified metabolites a measure of chemical diversity $\phi$ is calculated according to the one of the three formulas:

$$\emptyset_{Jaccard} = \sum_{a,b \in M} \sum_i w_a w_b (1 - F_i(a) \wedge F_i(b))$$

$$\emptyset_{Shannon} = -\sum_{k \in M} p_k \ln (p_k)$$

$$\emptyset_{Simpson} = \sum_{k \in M} p_k^2$$

wherein $M$ is the set of number of identified metabolites; $w_k$ is the weight for a given metabolite $k$ and is a transformed chromatographic peak height or area; $p_k$ is a set of metabolite weights converted to a probability distribution for a given metabolite $k$; and the $F_i$ are chemical fingerprinting functions.

11. The method according to claim 10, wherein the chemical fingerprinting functions comprise one or more of Morgan/Circular, Avalon, MACCS, PubChem, Topological-Torsion, CDK, and/or RDKit fingerprints.

12. The method according to claim 10, wherein the weight $w_k$ for a given metabolite k is a transformed chromatographic peak height or area using any combination of one or more of log transformation, intensity scaling, and linear regressions to correct for molecular ionizability.

13. The method according to claim 10, wherein all the molecules are displayed in a graph network where node lengths relate to chemical similarity.

14. The method according to claim 8, wherein the identified individual life forms of the holobiont are grouped into beneficial and pathogenic life forms.

15. The method according to any one of the preceding claims, wherein for any set of identified metabolites a plant defense-pathogen ratio as an indicator of the immunity or susceptibility of a plant to invading pathogens or other organisms is calculated according to the formula:

$$\frac{\psi_{int,PlantDefense}}{\psi_{int,Pathogen}}$$

wherein for a given set of metabolites M and weight $w_k$ for a given metabolite k

$$\psi_{int} = \sum_{k \in M} w_k$$

$\psi_{int,PlantDefense}$ is the Plant Defense Normalized Intensity of the set of identified metabolites that defend plants against pathogens and $\psi_{int,Pathogen}$ is the Plant Pathogen Normalized Intensity of the set of identified metabolites that are pathogenic to plants.

**FIGURE 1**

**FIGURE 2**

**FIGURE 3**

**FIGURE 4**

Europäisches
Patentamt

European
Patent Office

Office européen
des brevets

# EUROPEAN SEARCH REPORT

Application Number

EP 22 38 2921

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BONINI PAOLO ET AL: "Retip: Retention Time Prediction for Compound Annotation in Untargeted Metabolomics", ANALYTICAL CHEMISTRY, vol. 92, no. 11, 11 May 2020 (2020-05-11), pages 7515-7522, XP093029343, US ISSN: 0003-2700, DOI: 10.1021/acs.analchem.9b05765 * the whole document * * Introduction; page 7515 - page 7516 * * Software Implementation and Application Application of Retention Time Predictions with the External MS-FINDER Software; page 7519 - page 7520 * ----- | 1-15 | INV. G16C20/20 |
| X | IVANA BLA?ENOVI? ET AL: "Software Tools and Approaches for Compound Identification of LC-MS/MS Data in Metabolomics", METABOLITES, vol. 8, no. 2, 1 June 2018 (2018-06-01), page 31, XP055629597, ISSN: 2218-1989, DOI: 10.3390/metabo8020031 * the whole document * ----- | 1-15 | |
| X | US 2015/340216 A1 (KWIECIEN NICHOLAS W [US] ET AL) 26 November 2015 (2015-11-26) * the whole document * ----- -/-- | 1-15 | TECHNICAL FIELDS SEARCHED (IPC) G16C H01J G16B |

The present search report has been drawn up for all claims

| Place of search | Date of completion of the search | Examiner |
|---|---|---|
| The Hague | 8 March 2023 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 4 350 699 A1**

Europäisches
Patentamt

European
Patent Office

Office européen
des brevets

# EUROPEAN SEARCH REPORT

Application Number

EP 22 38 2921

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BAUERMEISTER ANELIZE ET AL: "Mass spectrometry-based metabolomics in microbiome investigations", NATURE REVIEWS MICROBIOLOGY, NATURE PUBLISHING GROUP, GB, vol. 20, no. 3, 22 September 2021 (2021-09-22), pages 143-160, XP037694345, ISSN: 1740-1526, DOI: 10.1038/S41579-021-00621-9 [retrieved on 2021-09-22] * the whole document * | 1-15 | |
| A | SANDRIN TODD R. ET AL: "Characterization of microbial mixtures by mass spectrometry", MASS SPECTROMETRY REVIEWS., vol. 37, no. 3, 16 May 2007 (2007-05-16), pages 321-349, XP093030030, US ISSN: 0277-7037, DOI: 10.1002/mas.21534 Retrieved from the Internet: URL:https://onlinelibrary.wiley.com/doi/full-xml/10.1002/mas.21534> * the whole document * | 1-15 | |

**TECHNICAL FIELDS SEARCHED (IPC)**

The present search report has been drawn up for all claims

| Place of search | Date of completion of the search | Examiner |
|---|---|---|
| The Hague | 8 March 2023 | Denoual, Matthieu |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 22 38 2921

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2023

| Patent document cited in search report | | Publication date | Patent family member(s) | | Publication date |
|---|---|---|---|---|---|
| US 2015340216 | A1 | 26-11-2015 | CN | 107077592 A | 18-08-2017 |
| | | | EP | 3123495 A1 | 01-02-2017 |
| | | | US | 2015340216 A1 | 26-11-2015 |
| | | | WO | 2015148941 A1 | 01-10-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MARGULIS.** Words as battle cries--symbiogene-sis and the new field of endocytobiology. *Biocience,* October 1990, vol. 40 (9), 673-7